# EUROPEAN PATENT APPLICATION

(11) **EP 4 817 684 A2**
(43) Date of publication of application: **07.10.2026**
(21) Application number: 26199953.6
(22) Date of filing: 05.10.2021
(51) Int. Cl.: A61P 27/02

(54) **IOL WITH DRUG DELIVERY DEVICES AND UNINTERRUPTED PCO BARRIER EDGE**

(30) Priority: 05.10.2020 US 202017062909; 10.11.2020 GB 202017747
(62) Document number(s) of the earlier application(s) in accordance with Art. 76 EPC: 21878391.8 / 4 225 210
(71) Applicant: SpyGlass Pharma, Inc., Irvine, CA 92618 (US); The Regents of the University of Colorado, A Body Corporate, Denver, CO 80203 (US)
(72) Inventor: CABLE, Craig Alan II, Aliso Viejo, 92656 (US); SUSSMAN, Glenn R, Aliso Viejo, 92656 (US); DENNEWILL, James R, Aliso Viejo, 92656 (US); KAHOOK, Malik Y, Denver, 80203 (US)
(74) Representative: Mewburn Ellis LLP

(57) **Abstract**

Ophthalmic implants including an IOL with haptics and drug delivery devices secured to the haptics are provided. The posterior-most extent of the drug delivery device is located anterior to the posterior edge of PCE barrier edge, of the IOL. In other configurations, the drug delivery devices are provided with PCO barrier edges.

## Description

### CROSS-REFERENCE

This application claims the benefit of U.S. Patent Application No. 17/062,909, filed October 5, 2020, and GB Application No. 2017747.3 filed November 10, 2020, which applications are incorporated herein by reference in their entirety for all purposes.

### FIELD OF THE DISCLOSURE

The present disclosure described below relates to the field of ophthalmic implants such as intraocular lenses.

### BACKGROUND OF THE DISCLOSURE

Intraocular lenses (IOL's) are artificial lenses for the eye that can be implanted to replace the natural lens of a patient's eye after the natural lens is removed. The natural lens might be removed because it is affected by cataracts, and the IOL can be implanted to provide clear vision and some degree of focusing to the patient. An intraocular lens may also be implanted in a patient without removing the natural lens (a phakic intraocular lens or PIOL), to correct extreme near-sightedness or far-sightedness. It is advantageous to administer some therapeutic agents to the eye, coincident with implantation of the IOL, to alleviate various side effects of the IOL or treat other conditions of the eye that might coexist with the conditions that lead to cataracts. Side-effects such as infection and inflammation, and conditions such as glaucoma, can be treated with therapeutic agents that may be incorporated into additional devices that may be secured to the IOL. In addition to IOLs, ocular implants not including lenses may be implanted to address various other conditions. Posterior capsular opacification (PCO) is a condition that affects some IOL implantations and occurs due to migration of lens epithelial cells into the region between the IOL and the posterior portion of the lens capsule. As these lens epithelial cells proliferate and differentiate in this region, the posterior portion of the lens capsule becomes opaque. This can cause visual impairment, sometimes referred to as a secondary cataract. This effect may be limited by the structure of the IOL itself, such as forming the IOL with a posterior surface and circumferential side wall surface joined with a sharply angular edge (typically referred to as a square edge). The posterior portion of the lens capsule collapses onto the IOL, and fuses to the IOL, and the fused join between the posterior portion of the lens capsule and the sharply angular edge of the IOL serves as a barrier to lens epithelial cells.

Previous patent applications, such as Kahook, et al., Ophthalmic Device For Drug Delivery, U.S. Pub. 2020/0022840 (Jan. 23, 2020) disclose various configurations of drug delivery devices to be used in conjunction with IOLs. Others have proposed placement of drug delivery devices on haptics. For those situations in which placement of the drug delivery devices on the haptics is preferred, we propose configurations that do not disrupt the square edge needed to block migration of lens endothelial cells to the back side of the IOL.

### SUMMARY

The devices and methods described below provide for placement of drug delivery devices on IOL haptics while preserving a PCO barrier edge around the entire periphery of the back side of the IOL. The configuration may be employed with IOLs and other ophthalmic implants. The configuration allows implantation of an IOL along with implantation of drug delivery devices to treat various conditions affecting implant patients while maintaining a PCO barrier which is effective around the entire periphery of the IOL. The device includes an IOL assembly comprising a lens, and a haptic. The IOL assembly is characterized by an anterior surface and a posterior surface, and a circumferential side wall, wherein the posterior surface joins the circumferential side wall in a sharply angular edge, where the sharply angular edge defines a plane of the sharply angular edge, and the sharply angular edge is functional as a PCO barrier edge. The drug delivery devices are disposed on the haptics and are configured and disposed on the haptic such that a posterior-most surface of each drug delivery device is anteriorly displaced from the plane. With this construction, the presence of the drug delivery devices on the haptics do not disrupt formation of a PCO barrier between the sharp edge and the posterior portion of the lens capsule.

In another configuration which preserves a PCO barrier edge, the posterior-most extent of the drug delivery device may extend posteriorly beyond the plane of the PCO barrier edge of the lens. In this configuration, the drug delivery device is configured with a posterior surface and a circumferential side wall adjacent the posterior side wall, and, like the PCO barrier edge of the IOL described above, the posterior surface of the drug delivery device joins the circumferential side wall of the drug delivery device in a sharply angular edge (also a posterior-lateral edge).

Additionally, other configurations of the drug delivery device described below provide for stable positioning of the IOL and drug delivery device within the capsular bag. The anterior surface of the drug delivery device is configured such that forces exerted on the drug delivery device by the capsular bag do not promote tilting of the IOL relative to the equatorial plane of the capsular bag, so that the optical axis of the IOL lens is not angled relative to the optical axis of the eye. The posterior surface may be a flat surface parallel to the plane of the IOL, or a curved surface bounded by an edge which lies in a plane parallel to the plane of the IOL.

In various aspects, provided herein is an ophthalmic implant **(11)** comprising: an IOL assembly **(12)** comprising a lens **(24),** and a haptic **(28),** said IOL assembly characterized by an anterior surface **(24A)** and a posterior surface **(24P),** and a circumferential side wall **(24C),** wherein the posterior surface joins the circumferential side wall in a sharply angular edge **(24E),** said sharply angular edge **(24E)** defining a plane **(P)** of the sharply angular edge **(24E);** and a drug delivery device **(30)** disposed on the haptic **(28),** said drug delivery device **(30)** configured and disposed on the haptic **(28)** such that a posterior-most surface **(30P)** of the drug delivery device **(30)** is anteriorly displaced from the plane **(P),** whereby the presence of the drug delivery device **(30)** on the haptic does **(28)** not disrupt formation of a PCO barrier. In some instances, the posterior-most surface **(30P)** of the drug delivery device **(30)** is anteriorly displaced from the plane **(P)** by 0.1 to 1 millimeters. In some instances, the haptic **(28)** is joined to the lens **(24)** at a joint, and the posterior-most extent of the joint between the haptic and the lens is displaced/offset anteriorly from the sharply angular edge **(24E).** In some instances, the haptic is joined to the lens **(24)** at a joint, and the posterior-most extent of the joint is displaced/offset anteriorly from the sharply angular edge, whereby the presence of the haptic does not disrupt formation of a PCO barrier between the IOL sharp edge and the posterior portion of the capsule. In some instances, the sharply angular edge is characterized by an inside angle and a corner radius, between the posterior panel surface and the circumferential side wall of the posterior panel is in the range of 80° to 100°, and the corner radius of the sharply angular edge is less than 500 µm, and preferably less than 60 µm.

In various aspects, provided herein is a method of treating a patient, said method comprising the steps of: providing an ophthalmic implant comprising: an IOL assembly **(12)** comprising a lens **(24),** and a haptic **(28),** said IOL assembly characterized by an anterior surface **(24A)** and a posterior surface **(24P),** and a circumferential side wall **(24C),** wherein the posterior surface joins the circumferential side wall in a sharply angular edge **(24E),** said sharply angular edge defining a plane **(P)** of the sharply angular edge; and a drug delivery device **(30)** disposed on the haptic **(38),** said drug delivery device **(30)** configured and disposed on the haptic **(28)** such that a posterior-most surface of the drug delivery device is anteriorly displaced from the plane **(P),** whereby the presence of the drug delivery device on the haptic does not disrupt formation of a PCO barrier; wherein the drug delivery device contains a therapeutic agent effective to treat glaucoma, macular degeneration, uveitis, posterior capsular opacification, infection or inflammation of the eye; and implanting said ophthalmic implant within a capsular bag of an eye of the patient; and allowing the capsular bag to approximate the IOL, and form a PCO barrier between the posterior portion of the capsular bag and the sharply angular edge. In some instances, the posterior-most surface of the drug delivery device is anteriorly displaced from the plane **(P)** by 0.1 to 1.0 millimeters. In some instances, the haptic is joined to the lens **(24)** at a joint, and the posterior-most extent of the joint between the haptic and the lens is displaced/offset anteriorly from the sharp edge. In some instances, the haptic is joined to the lens **(24)** at a joint, and the posterior-most extent of the joint is displaced/offset anteriorly from the sharp edge, whereby the presence of the haptic does not disrupt formation of a PCO barrier between the IOL sharp edge and the posterior portion of the capsule.

In various aspects, provided herein is an ophthalmic implant **(11)** comprising: an IOL assembly **(12)** comprising a lens **(24),** and a haptic **(28),** said IOL assembly characterized by an anterior surface and a posterior surface, and a circumferential side wall **(24C),** wherein the posterior surface joins the circumferential side wall in a sharply angular edge **(24E),** said sharply angular edge defining a plane **(P)** of the sharply angular edge; and a drug delivery device disposed on the haptic, said drug delivery device having a posterior surface **(33P),** and a circumferential side wall **(33C),** wherein the posterior surface joins the circumferential side wall in a sharply angular edge **(33E).** In some instances, the drug delivery device comprises a posterior panel **(33)** and an anterior panel **(36)** joined by a pair of posts **(35),** with an aperture **(34)** bounded by said posterior panel **(33),** anterior panel **(36)** and posts **(35),** and the haptic is disposed within the aperture to secure the drug delivery device to the haptic. In some instances, a portion of the circumferential side wall **(33C)** of the posterior panel **(33)** immediately abuts a portion of the side wall **(24C)** of the IOL **(24).** In some instances, the circumferential side wall **(33C)** of the posterior panel **(33)** is radially, outwardly displaced from the side wall **(24C)** of the IOL **(24).** In some instances, the drug delivery device **(30)** is configured and disposed on the haptic **(28)** such that the sharply angular edge **(33E)** of the drug delivery device **(30)** is anteriorly displaced from the plane **(P).** In some instances, the drug delivery device **(30)** is configured and disposed on the haptic **(28)** such that the sharply angular edge **(33E)** of the drug delivery device **(30)** is posteriorly displaced from the plane **(P).** In some instances, the drug delivery device **(30)** is configured and disposed on the haptic **(28)** such that the sharply angular edge **(33E)** of the drug delivery device **(30)** is co-planar with the plane **(P).**

In various aspects, provided herein is a method of treating a patient, said method comprising the steps of: providing an ophthalmic implant comprising: an IOL assembly **(12)** comprising a lens **(24),** and a haptic **(28),** said IOL assembly characterized by an anterior surface and a posterior surface, and a circumferential side wall **(24C),** wherein the posterior surface joins the circumferential side wall in a sharply angular edge **(24E),** said sharply angular edge defining a plane **(P)** of the sharply angular edge; and a drug delivery device configured to be secured to the haptic, said drug delivery device having a posterior surface **(33P),** and a circumferential side wall **(33C),** wherein the posterior surface joins the circumferential side wall in a sharply angular edge **(33E);** wherein the drug delivery device contains a therapeutic agent effective to treat glaucoma, macular degeneration, uveitis, posterior capsular opacification, infection or inflammation of the eye; and securing the drug delivery device to the haptic; implanting said ophthalmic implant within a capsular bag of an eye of the patient; and allowing the capsular bag to approximate the IOL, and form a PCO barrier between the posterior portion of the capsular bag and the sharply angular edge of the IOL and form a PCO barrier between the capsular bag and the sharply angular edge of the posterior panel. In some instances, the drug delivery device comprises posterior panel **(33)** and an anterior panel **(36)** joined by a pair of posts **(35),** with an aperture **(34)** bounded by said posterior panel **(33),** anterior panel **(36)** and posts **(35);** and the method further comprises securing the drug delivery device **(30)** on the haptic **(28)** by inserting the haptic **(28)** into the aperture **(34).** In some instances, securing the drug delivery device **(30)** on the haptic **(28)** such that a portion of the circumferential side wall **(33C)** of the posterior panel **(33)** immediately abuts a portion of the side wall **(24C)** of the IOL **(24).** In some instances, securing the drug delivery device **(30)** on the haptic **(28)** such that the circumferential side wall **(33C)** of the posterior panel **(33)** is radially, outwardly displaced from the side wall **(24C)** of the IOL **(24),** leaving a radial gap between the PCO barrier edge of the IOL and the PCO barrier edge of the posterior panel **(33).** In some instances, such method further comprises the steps of: securing the drug delivery device **(30)** on the haptic **(28)** such that the sharply angular edge **(33E)** of the drug delivery device **(30)** is anteriorly displaced from the plane **(P).** In some instances, securing the drug delivery device **(30)** on the haptic **(28)** such that the sharply angular edge **(33E)** of the drug delivery device **(30)** is posteriorly displaced from the plane **(P).** In some instances, securing the drug delivery device **(30)** on the haptic **(28)** such that the sharply angular edge **(33E)** of the drug delivery device **(30)** is co-planar with the plane **(P).**

In various aspects, provided herein is an ophthalmic implant **(11)** comprising: an IOL assembly **(12)** comprising a lens **(24),** and a haptic **(28),** the IOL assembly characterized by an anterior surface and a posterior surface, and a circumferential side wall **(24C),** wherein the posterior surface joins the circumferential side wall in a sharply angular edge **(24E),** the sharply angular edge defining a plane **(P)** of the sharply angular edge; and a drug delivery device disposed on the haptic, the drug delivery device having a posterior surface **(33P),** and a circumferential side wall **(33C),** wherein the drug delivery device on the haptic does not disrupt formation of a PCO barrier. In some instances, the drug delivery device **(30)** is configured and disposed on the haptic **(28)** such that the sharply angular edge **(33E)** of the drug delivery device **(30)** is anteriorly displaced from the plane **(P).** In some instances, the drug delivery device **(30)** is configured and disposed on the haptic **(28)** such that the sharply angular edge **(33E)** of the drug delivery device **(30)** is posteriorly displaced from the plane **(P).** In some instances, the drug delivery device **(30)** is configured and disposed on the haptic **(28)** such that the sharply angular edge **(33E)** of the drug delivery device **(30)** is co-planar with the plane **(P).** In some instances, the drug delivery device comprises a posterior panel **(33)** and an anterior panel **(36)** joined by a pair of posts **(35),** with an aperture **(34)** bounded by the posterior panel **(33),** anterior panel **(36)** and posts **(35),** and the haptic is disposed within the aperture to secure the drug delivery device to the haptic. In some instances, a portion of the circumferential side wall **(33C)** of the posterior panel **(33)** immediately abuts a portion of the side wall **(24C)** of the IOL **(24).** In some instances, the circumferential side wall **(33C)** of the posterior panel **(33)** is radially, outwardly displaced from the side wall **(24C)** of the IOL **(24).**

In various aspects, provided herein is an ophthalmic implant (11) comprising: an IOL assembly **(12)** comprising a lens **(24),** and a haptic **(28),** the IOL assembly characterized by an anterior surface and a posterior surface, and a circumferential side wall **(24C);** and the haptic **(28)** is joined to the lens via a haptic base **(28B)** having a shape and size configured for acceptance of a drug delivery device **(30);** a drug delivery device **(30)** configured for disposition on the haptic **(28),** the drug delivery device **(30)** having an aperture **(34)** configured to accept the haptic base **(28B),** the aperture **(34)** being keyed to the shape and size of the haptic base **(28B)** in a configuration which inhibits rotation of the drug delivery device **(30)** about the haptic base **(28B);** such that, in an assembled configuration, the drug delivery device **(30)** is disposed on the haptic base **(28B),** with the haptic base **(28B)** occupying the aperture **(34).** In some instances, the drug delivery device **(30)** comprises a posterior panel **(33)** and an anterior panel **(36)** joined by a pair of posts **(35),** with an aperture **(34)** bounded by the posterior panel **(33),** anterior panel **(36)** and posts **(35).**

In various aspects, provided herein is an ophthalmic implant **(11)** comprising: an IOL assembly **(12)** comprising a lens **(24),** and a haptic **(28),** the IOL assembly characterized by an anterior surface and a posterior surface, and a circumferential side wall **(24C);** and the haptic **(28)** is joined to the lens **(12)** via a haptic base **(28B)** having a shape and size configured for acceptance of a drug delivery device **(30);** a drug delivery device **(30)** configured for disposition on the haptic, the drug delivery device **(30)** having an aperture **(34)** configured to accept the haptic base **(28B),** the aperture **(34)** being keyed to the shape and size of the haptic base **(28B)** in a configuration which inhibits rotation of the drug delivery device **(30)** about the haptic base **(28B);** such that, in an assembled configuration, the drug delivery device **(30)** is disposed on the haptic base **(28B),** with the haptic base **(28B)** occupying the aperture **(34).** In some instances, the drug delivery device **(30)** comprises a posterior panel **(33)** and an anterior panel **(36)** joined by a pair of posts **(35),** with an aperture **(34)** bounded by the posterior panel **(33),** anterior panel **(36)** and posts **(35).** In some instances, the drug delivery device **(30)** comprises an anterior panel **(36)** with an anterior surface **(36A)** and an anterior surface edge **(36E),** and the anterior surface edge **(36E)** lies entirely in a plane **(A)** which is parallel to a major plane of the IOL lens **(B).** In some instances, the anterior surface **(36A)** is planar. In some instances, the anterior panel **(36)** is characterized by a circumferential dimension **(40),** a width **(42),** and a radial dimension **(41)** and the circumferential dimension **(40)** is larger than the radial dimension **(41).**

In various aspects, provided herein is an ophthalmic implant **(11)** comprising: an IOL assembly **(12)** comprising a lens **(24),** and a haptic **(28),** the IOL assembly characterized by an anterior surface and a posterior surface, and a circumferential side wall **(24C);** the haptic **(28)** is joined to the lens via a haptic base **(28B)** having a shape and size configured for acceptance of a drug delivery device **(30);** a drug delivery device **(30)** configured for disposition on the haptic **(28),** the drug delivery device **(30)** having an aperture **(34)** configured to accept the haptic base **(28B);** the haptic further comprising a first retaining structure, configured to inhibit outward migration of the drug delivery device **(30)** from the haptic base; wherein in an assembled configuration, first retaining structure **(37, 36, 39)** obstructs the drug delivery device **(30)** and inhibits outward migration of the drug delivery device **(30)** from the haptic base **(28B).** In some instances, the first retaining structure comprises a detent or shoulder on the haptic, configured to inhibit with outward movement of the drug delivery device **(30)** along the haptic **(28).** In some instances, the first retaining structure comprises a notch in the haptic base **(28B);** and the drug delivery device **(30)** comprises a second retaining feature comprising a post **(35)** configured to occupy the notch. In some instances, the drug delivery device **(30)** comprises a posterior panel **(33)** and an anterior panel **(36)** joined by a pair of posts **(35),** with an aperture **(34)** bounded by the posterior panel **(33),** anterior panel **(36)** and the posts **(35);** and the drug delivery device **(30)** is disposed over the haptic with one of the posts **(35)** disposed within the notch. In some instances, the drug delivery device **(30)** is configured and disposed on the haptic **(28)** such that the sharply angular edge **(33E)** of the drug delivery device **(30)** is anteriorly displaced from the plane **(P).** In some instances, the drug delivery device **(30)** is configured and disposed on the haptic **(28)** such that the sharply angular edge **(33E)** of the drug delivery device **(30)** is posteriorly displaced from the plane **(P).** In some instances, the drug delivery device **(30)** is configured and disposed on the haptic **(28)** such that the sharply angular edge **(33E)** of the drug delivery device **(30)** is co-planar with the plane **(P).** In some instances, the posterior-most surface **(30P)** of the drug delivery device **(30)** is anteriorly displaced from the plane **(P)** by 0.1 to 1 millimeters.

In various aspects, provided herein is an ophthalmic implant **(11)** comprising: an IOL assembly **(12)** comprising a lens **(24),** and a haptic **(28),** the IOL assembly characterized by an anterior surface **(24A)** and a posterior surface **(24P),** and a circumferential side wall **(24C),** wherein the posterior surface joins the circumferential side wall in a sharply angular edge **(24E),** the sharply angular edge **(24E)** defining a plane **(P)** of the sharply angular edge **(24E);** and a drug delivery device **(30)** disposed on the haptic **(28),** the drug delivery device **(30)** configured and disposed on the haptic **(28)** such that a posterior-most surface **(30P)** of the drug delivery device **(30)** is anteriorly displaced from the plane **(P),** whereby the presence of the drug delivery device **(30)** on the haptic does **(28)** not disrupt formation of a PCO barrier. In some instances, the posterior-most surface **(30P)** of the drug delivery device **(30)** is anteriorly displaced from the plane **(P)** by 0.1 to 1 millimeters. In some instances, the haptic **(28)** is joined to the lens **(24)** at a joint, and the posterior-most extent of the joint between the haptic and the lens is displaced/offset anteriorly from the sharply angular edge **(24E).** In some instances, the haptic is joined to the lens **(24)** at a joint, and the posterior-most extent of the joint is displaced/offset anteriorly from the sharply angular edge, whereby the presence of the haptic does not disrupt formation of a PCO barrier between the IOL sharp edge and the posterior portion of the capsule. In some instances, the sharply angular edge is characterized by an inside angle and a corner radius, between the posterior panel surface and the circumferential side wall of the posterior panel is in the range of 80° to 100°, and the corner radius of the sharply angular edge is less than 500 µm, and preferably less than 60 µm. In some instances, the drug delivery device comprises one or more therapeutic agents. In some instances, the one or more therapeutic agents comprises a beta blocker, an alpha agonist, a ROCK Inhibitor, an adenosine receptor agonist, a carbonic anhydrase inhibitor, an adrenergic or cholinergic receptor activating agent, a prostaglandin analogue, a steroid, an aptamer, an antibody, a complement factor, an anti-oxidant, an anti-inflammatory agent, an anti-proliferative agents, an anti-mitotic agents, an antibiotic, or a combination thereof. In some instances, the one or more therapeutic agents comprises or consists of a prostaglandin analogue.

In various aspects, provided herein is a method of treating an eye disease in a patient in need thereof, the method comprising: implanting an ophthalmic implant according to the present disclosure within a capsular bag of the eye of the patient, thereby treating the eye disease in the patient. In some instances, such method further comprises allowing the capsular bag to approximate the IOL. In some instances, such method further comprises forming a PCO barrier between the posterior portion of the capsular bag and the sharply angular edge. In some instances, the eye disease comprises glaucoma, wet macular degeneration, dry macular degeneration, uveitis, an infection or inflammation of the eye, or a combination thereof. In some instances, the implanting comprises: folding or rolling the ophthalmic implant into a small diameter configuration into an injector; inserting the injector through a small slit between the cornea and sclera of the eye; and pushing the ophthalmic implant from the injector into a tissue of the eye.

### BRIEF DESCRIPTION OF THE DRAWINGS

The novel features of the invention are set forth with particularity in the appended claims. A better understanding of the features and advantages of the present disclosure will be obtained by reference to the following detailed description that sets forth illustrative embodiments, in which the principles of the invention are utilized, and the accompanying drawings of which:
**Figures 1** and **2** illustrate the environment of use of an ophthalmic implant system for drug delivery.
**Figure 3** is a view of the IOL and drug delivery devices, showing the posterior aspect of the device.
**Figure 4** is a view of the IOL and drug delivery devices, showing the anterior aspect of the device.
**Figure 5** illustrates the intraocular lens and drug delivery devices, illustrating the anterior offset of the haptics and drug delivery devices.
**Figure 6** illustrates the sharply angular edge of the IOL.
**Figures 7** and **8** illustrate an IOL and drug delivery device combination in which the drug delivery device posterior is provided with a PCO barrier edge distinct from the PCO barrier edge of the IOL.
**Figure 9** shows the drug delivery device similar to that of **Figure 8** with the addition of additional panels or blocks depending from an anterior panel of the drug delivery device.
**Figure 10** illustrates a configuration in which the plane **(D)** is posteriorly displaced from the plane **(P)** defined by the PCO barrier edge **24E** of the IOL.
**Figure 11** illustrates a configuration of the drug delivery device and the haptic configured to avoid twisting of the drug delivery device about the haptic.
**Figures 12** and **13** illustrate a means of preventing outward movement of the drug delivery device on the haptic, and features of the drug delivery device which prevent or limit twisting of the drug delivery device within the capsular bag.

### DETAILED DESCRIPTION OF THE DISCLOSURE

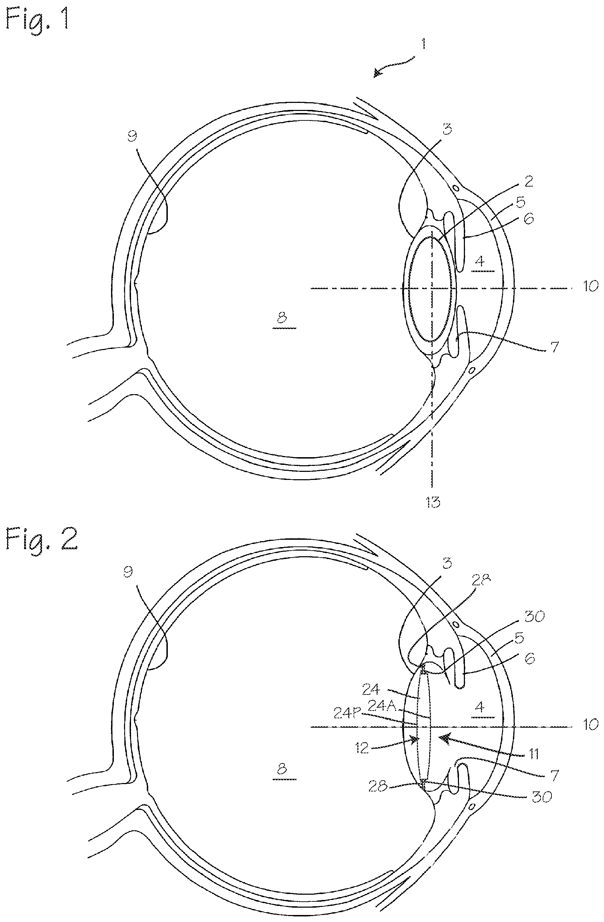

**Figures 1** and **2** illustrate placement and use of an IOL (or other ophthalmic implant systems) in the eye of a patient. The eye **1** includes a lens **2** (the natural lens of the eye) and lens capsular bag **3,** and the anterior chamber **4** which includes the cornea **5** and iris **6** and aqueous humour filling the space between the cornea and the iris, and a posterior chamber **7** between the iris and the capsular bag. The posterior cavity/vitreous body **8** is the large space between the lens and the retina **9.** The natural lens **2** of the eye is characterized by an optical axis **10.** The capsular bag is characterized by an equatorial plane **13,** which is generally perpendicular to the optical axis. (In the following description of the ophthalmic implant and/or intraocular lens system, the terms posterior and anterior will be used in relation to the anatomy of the eye, in which the cornea is anterior, and the retina is posterior.)

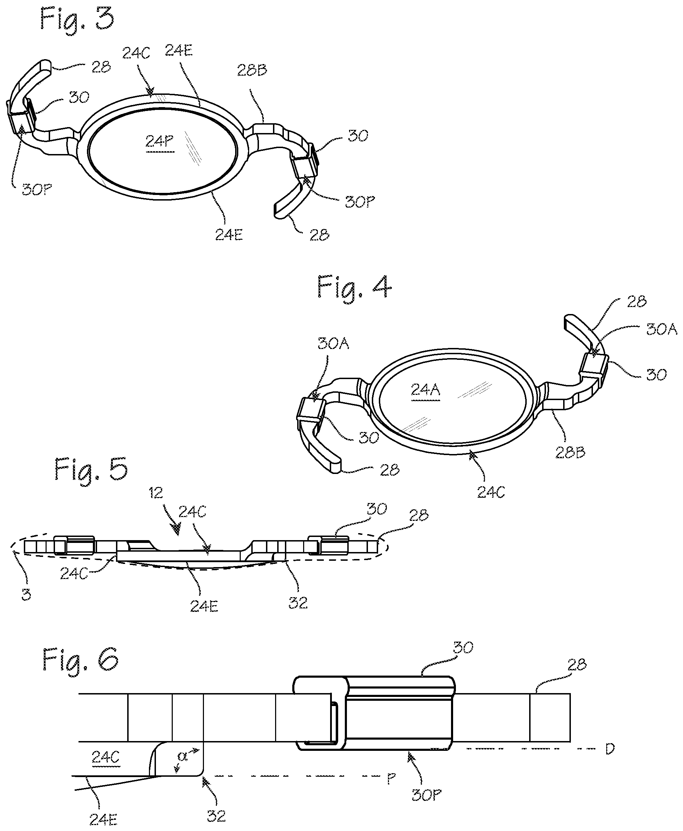

**Figure 2** illustrates a placement of the IOL system (or other ophthalmic implant system) **11** in the eye including the intraocular lens assembly **12** and drug delivery device **30** which is implanted in the capsular bag in conjunction with the intraocular lens assembly **12.** As shown in **Figure 2****,** and in more detail in **Figures 3** through **5****,** the drug delivery device **30** is disposed on the haptics **28.** The intraocular lens assembly **12** includes a lens **24** and one or more haptics **28,** and one or more drug delivery devices **30.** The intraocular lens assembly **12** is characterized by an anterior surface **24A** and a posterior surface **24P** and a circumferential side wall surface **24C.** Other ophthalmic implants such as a capsular tension ring, or a capsular scaffold for holding the drug delivery devices in place may take the place of the IOL. The haptics may be any form of haptic, such as C-loops or J-loops.

**Figure 3** is a view of the IOL **12** and drug delivery devices **30,** showing the posterior aspect of the device, including the posterior surface **24P** of the lens **24,** and the sharply angular posterior-lateral edge **24E** of the IOL, which may be the edge of the lens proper, or the edge of a frame surrounding the lens. The joint **31** between the haptic and the lens is preferably displaced/offset anteriorly from the sharp edge, to retain the PCO edge entirely around the periphery of the posterior side of the lens. (Typically, the lens and haptics will be integrally formed, and this "joint" refers to the transition between the lens and the haptic.) A drug delivery device **30** is disposed on one or both haptics **28** (preferably both), secured in any suitable manner, such as a friction fit of the tubular (rectangular or cylindrical) drug delivery device slipped over the haptic. The drug delivery device is characterized by a posterior surface **30P,** facing posteriorly just as the posterior surface **24P** of the lens does. The posterior surface **30P** is also displaced/offset anteriorly from the posterior-lateral edge **24E,** to avoid disruption of the fusion of the posterior capsule **3** to the posterior-lateral edge **24E.** For completeness, **Figure 4** is a view of the IOL and drug delivery devices, showing the anterior aspect of the device, including the anterior surface **24A** of the lens **24,** each haptic **28,** and the drug delivery devices **30** secured to the haptics, comprising an anterior surface **30A.** The front edge of the IOL, at which point the anterior surface **24A** meets the circumferential side wall surface of the IOL, may be a sharply angular edge comparable to the edge **24E** on the posterior side of the IOL, or may be rounded or configured to obtain desired optical characteristics.

**Figure 5** illustrates the intraocular lens assembly **12,** including the lens **24,** one or more haptics **28,** and one or more drug delivery devices **30.** As shown in **Figure 5****,** the sharp edge **24E** between the posterior surface **24P** of the IOL and circumferential side wall surface **24C** is an edge that circumscribes the lens **24** (preferably entirely circumscribes the lens), and establishes a plane **(P)** in which the sharp edge lies. The posterior-most extent of the drug delivery devices and optionally, the joint between the haptic and the lens is displaced anteriorly from this plane, not extending posteriorly to the plane **(P)** established by the sharp posterior edge of the IOL. In **Figure 6****,** the plane of the sharp posterior edge is marked as plane **(P).** The posterior extent of the drug delivery devices attached to the haptics is limited (and, optionally, the haptic joint), for example, by an offset represented by plane **D.** The offset is sufficient to avoid disruption of the fusion of the posterior portion of the capsule with the sharp angular edge **24E,** and may be 0.1 to 1.0 mm. The sharply angular edge **24E** may be any edge configured to function as a PCO barrier edge, and may be characterized by, for example, and inside angle α of 80-100°, and a corner radius **32** of less than 500 µm, and preferably less than 60 µm. This is illustrated in **Figure 6****.**

As shown in **Figure 5****,** the natural lens capsule **3** (shown in phantom) has collapsed onto the IOL, and, along the line at which the capsule meets the sharp edge **24E,** and this forms an effective barrier against migration of the lens endothelial cells from the anterior chamber into the space posterior to the IOL, between the IOL posterior surface and the capsule posterior surface.

The drug delivery devices may be provided in many configurations. As illustrated in the figures, the drug delivery devices may be configured as a collar or sleeve with a through-hole, which can be slipped over the haptic and retained with a friction fit or detent arrangement. The drug delivery devices may comprise a polymer matrix with therapeutic agent(s) embedded in the matrix, or it may be comprised of a therapeutic agent in an erodible form. The matrix preferably comprises silicone, though other suitable matrix material may be used. The entirety of the drug delivery device may comprise the matrix material loaded with therapeutic agent, or an erodible therapeutic agent, or the matrix material loaded with therapeutic agent, or an erodible therapeutic agent may be limited to portions of the drug delivery device, while the remaining portions are provided for structure.

In use, the IOL assembly of **Figures 3** through **6** with the drug delivery device configured and disposed on the haptic such that a posterior-most surface of the drug delivery device will be anteriorly displaced from the plane **(P)** once installed so that the presence of the drug delivery device on the haptic does not disrupt formation of a PCO barrier with the PCO barrier edge of the IOL lens when implanted within a capsular bag of an eye of the patient; and thereafter the capsular bag will approximate the IOL, and form a PCO barrier between the posterior portion of the capsular bag and the sharply angular edge uninterrupted by the posterior of the drug delivery device.

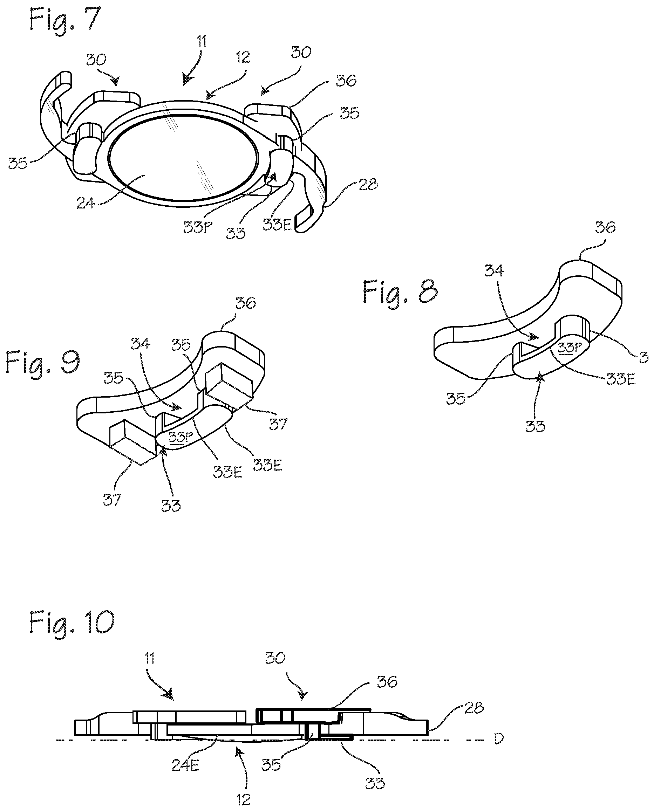

**Figures 7** and **8** illustrate an IOL **12** and drug delivery device **30** assembled, in which the drug delivery device posterior is provided with a PCO barrier edge distinct from the PCO barrier edge of the IOL. As shown in **Figure 7****, the** IOL system **11** includes the IOL **12** with haptics **28** extending from the lens **24.** The drug delivery device **30** is disposed on the haptic, and, in this configuration, includes a posterior panel **33** with a posterior surface **33P** which may or may not extend posteriorly beyond the plane (P) of the PCO barrier edge (shown in **Figures 5** and **6****),** and an oblong aperture **34** which accommodates a portion of the haptic nearest the rim of the IOL lens which is oblong and mated to the oblong aperture. Two posts **35** extend anteriorly from the posterior panel **33** to join with an anterior panel **36** which is disposed on the anterior side of the haptic **28,** forming the aperture **34** which is bounded by the posterior panel **33,** anterior panel **36** and posts **35.** As appears from **Figure 8****,** the posterior surface **33P** of posterior panel **33** of the drug delivery device is joined to the side wall **33C** of the posterior panel with a sharply angular edge **33E.** This sharply angular edge may be similar to that of the sharply angular edge of the IOL, formed at the junction of the posterior surface **33P** and the circumferential side wall **33C** of the posterior side wall. The sharply angular edge **33E** of the posterior panel may define a plane **(D)** which may be posterior to, or anterior to, or co-planar with, the plane **(P)** defined by the PCO barrier edge **24E** of the IOL. Thus, the drug delivery device **30** is configured and disposed on the haptic **28** such that the sharply angular edge **33E** of the drug delivery device **30** is anteriorly displaced from the plane **(P),** posteriorly displaced from the plane **(P),** or co-planar with the plane **(P).** **Figure 10** illustrates a configuration in which the plane **(D)** is posteriorly displaced from the plane **(P)** defined by the PCO barrier edge **24E** of the IOL.

The sharply angular edge **33E** of the posterior panel **33** preferably extends entirely around the posterior panel and side wall of the posterior panel. The sharply angular edge **33E** of the posterior panel **33,** and the circumferential side wall **33C** may immediately abut the circumferential side wall **24C** of the IOL, or the sharply angular edge **33E** of the posterior panel **33,** and the circumferential side wall **33C** may be spaced from the circumferential side wall **24C,** leaving a small radial gap between the PCO barrier edge of the IOL and the PCO barrier edge of the posterior panel **33,** such that the circumferential side wall **33C** of the posterior panel **33** is radially, outwardly displaced from the side wall **24C** of the IOL **24.**

If the plane **(D)** is coplanar with the plane **(P),** and the posterior panel directly abuts the IOL side wall, the area of the posterior panel directly abutting the IOL side wall may be provided with an edge that is not sharply angular, and the PCO barrier edge and remaining edge of the posterior panel will provide a sharply angular edge around entire periphery of the combined IOL and drug delivery device.

**Figure 8** shows the drug delivery device **30** of **Figure 7** in isolation, showing the aperture **34,** posts **35** and posterior panel **33** and the anterior panel **36.** **Figure 9** shows the drug delivery device **30** similar to that of **Figure 8** showing the apertures **34,** posts **35** and posterior panel **33** and the anterior panel **36,** with the addition of additional side panels or blocks **37.** The side panels may extend posteriorly from the anterior panel, or they may be fixed to the posts. The portion of this drug delivery device which comprises a matrix material loaded with therapeutic agent, or an erodible therapeutic agent may include one or more, or all, of the anterior panel, posts, posterior panel, or side panel(s).

In use, the IOL assembly and drug delivery device configurations of **Figures 7, 8** and **9** may be implanted within the capsular bag of an eye of the patient, and the capsular bag may then approximate the IOL, and form a PCO barrier between the posterior portion of the capsular bag and the sharply angular edge of the IOL and form a PCO barrier between the capsular bag and the sharply angular edge of the posterior panel. The drug delivery device **30** will be secured on the haptic **28** such that a portion of the circumferential side wall **33C** of the posterior panel **33** immediately abuts a portion of the side wall **24C** of the IOL **24,** or such that the circumferential side wall **33C** of the posterior panel **33** is radially, outwardly displaced from the side wall **24C** of the IOL **24,** leaving a radial gap between the PCO barrier edge of the IOL and the PCO barrier edge of the posterior panel **33.**

This may be accomplished by securing the drug delivery device **30** on the haptic **28** such that the sharply angular edge **33E** of the drug delivery device **30** is anteriorly displaced from the plane **(P),** such that the sharply angular edge **33E** of the drug delivery device **30** is posteriorly displaced from the plane **(P),** or such that the sharply angular edge **33E** of the drug delivery device 30 is co-planar with the plane **(P).** In some instances, the drug delivery device **30** is configured on the haptic **28** such that the sharply angular edge **33E** of the drug delivery device **30** is anteriorly displaced from the plane **(P).**

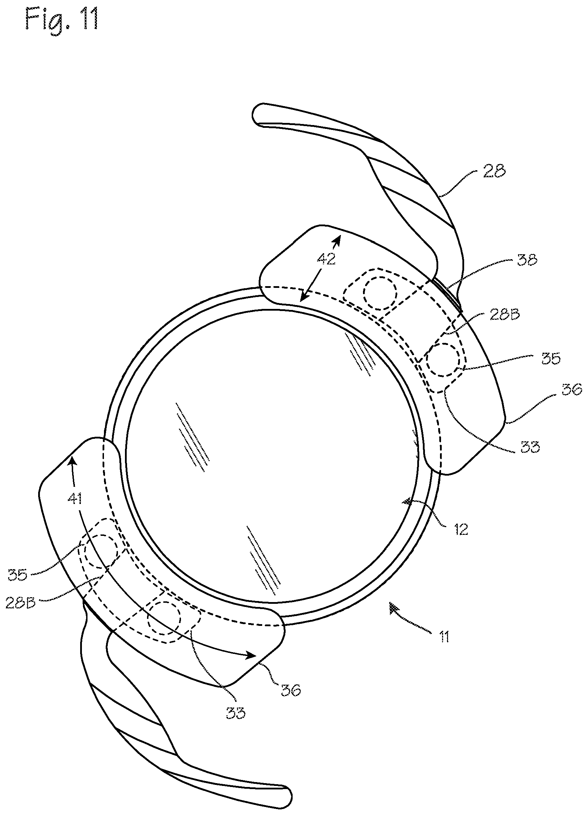

Various configurations of the drug delivery device may be employed to ensure that the IOL assembly **11,** once implanted, remains in the preferred anterior/posterior position within the capsular bag, and remains in the desired orientation with the plane of the lens **24** perpendicular to the optical axis **10** of the eye, and, correspondingly, coplanar with the equatorial plane of the capsular bag, or in a plane parallel to the equatorial plane of the capsular bag. **Figure 11** illustrates a configuration of the drug delivery device **30** and the haptic **28.** To avoid twisting of the drug delivery device about the haptic, the aperture through the drug delivery device (between the posterior panel and the anterior panel and connecting posts) is keyed to the shape of the base of the haptic, in any suitable manner and any suitable means, to inhibit or prevent rotation of the drug delivery device about the base of the haptic. In **Figure 11****,** this is accomplished with a haptic base **28B** having a square or rectangular cross section, and a drug delivery device having a matching square or rectangular aperture sized to accommodate the haptic base. Thus, the drug delivery device is configured for disposition on the haptic, and the aperture is configured to accept the haptic base, and the aperture is correspondingly keyed to the shape and size of the haptic base in a configuration which inhibits rotation of the drug delivery device about the haptic base. In the assembled configuration, the drug delivery device is disposed on the haptic base, with the haptic base occupying the aperture. Any other configuration, in which the aperture is keyed to the haptic base, may be employed, such as an elliptical haptic base paired with an elliptical aperture, or a hexagonal haptic base paired with a hexagonal aperture in the drug delivery device. Thus, various means for preventing twisting of the drug delivery device about the haptic or haptic base may be employed.

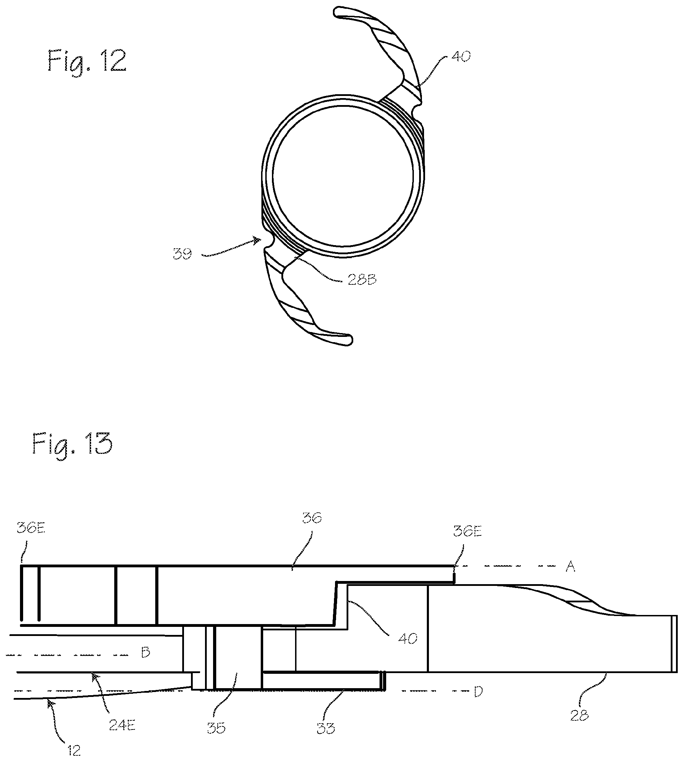

Configurations for preventing radially outward movement of the drug delivery device on the haptic may also be employed. As shown in **Figure 11** for example, the haptic may include a detent ridge **38** protruding from the haptic, with an optional corresponding detent accepting groove on the interior of the drug delivery device aperture. The drug delivery device may, once in place over the haptic base, impinge or abut the detent, thus preventing movement of the drug delivery device over the haptic. **Figures 12** and **13** illustrate an embodiment of the haptic and drug delivery device, in which the haptic has a notch **39** disposed on the side of the haptic base **28B.** The notch combined with a configuration of the post of the drug delivery device which fits within the notch provides an interference fit between the drug delivery device and the haptic. Once the drug delivery device is stretched over the haptic base, a post of the drug delivery device will fit within the notch, and the interference fit between the notch and the post will prevent outward migration of the drug delivery device.

**Figures 12** and **13** illustrate another means of preventing outward movement, which includes a shoulder rising anteriorly from the haptic, combined with a shoulder on the anterior panel or plate of the drug delivery device. Once the drug delivery device is stretched over the haptic base, the drug delivery device will impinge on the shoulder and interfere with the shoulder and will prevent outward migration of the drug delivery device.

**Figure 13** also illustrates features of the drug delivery device which prevent or limit twisting of the drug delivery device with the capsular bag. The anterior panels are configured to limit the potential for twisting of the IOL relative the equator, or the plane established by the equator, of the capsular bag. Referring to **Figure 13****,** the anterior panel **36** has planar anterior surface **36A** in which the entire edge **36E** lies in a plane **A,** or a curved anterior surface in which the entire edge **36E** lies in a plane **A,** and this planar surface or edge plane is disposed in a plane **A** which is parallel to the major plane of the IOL lens **B,** and, when properly implanted, the equatorial plane of the capsular bag (item **13** in **Figure 1****).** The anterior panel may have a large lateral or circumferential dimension compared to the radial dimension **(****Figure 11****)** and the anterior surface of the anterior panel is preferably flat and aligned in a plane parallel to the plane of the IOL. With this configuration, collapse of the capsular bag upon the IOL and the drug delivery device will be unlikely to exert forces on the IOL and drug delivery device which may twist or tilt the IOL within the capsular bag.

The IOL may be implanted by folding or rolling it into a small diameter configuration in an injector, inserting the injector through a small slit between the cornea and sclera, and pushing the IOL from the injector. Upon ejection from the injector, the IOL unfurls within the capsular bag and the haptics open and expand to the extent permitted by the confines of the capsular bag. The capsular bag will eventually collapse upon the IOL, and the posterior portion of the capsule will approximate the surfaces of the IOL, and, in particular, the sharp edge **24E.** The tight join between the capsule and the sharp edge inhibits migration of lens epithelial cells to the region between the posterior portion of the capsule and the posterior surface of the IOL. The drug delivery device may be secured to the haptic upon manufacture, or it may be secured by the surgeon immediately before implantation, immediately after implantation, or long after implantation (for example as a replacement for an original drug delivery device which has been depleted).

In each embodiment described, the drug delivery device can be configured to deliver various therapeutic agents to treat various conditions. Brimonidine, latanoprost, timolol, pilocarpine, brinzolamide and other drugs in the general categories of beta blockers, alpha agonists, ROCK Inhibitors, adenosine receptor agonists, carbonic anhydrase inhibitors, adrenergic and cholinergic receptor activating agents, and prostaglandin analogues may be incorporated into the drug delivery devices to treat glaucoma. Aflibercept, bevacizumab, pegaptanib, ranibizumab, steroids, and aptamers may be incorporated into the drug delivery devices to treat wet macular degeneration. Complement factors, antioxidants and anti-inflammatory agents may be incorporated into the drug delivery devices to treat dry macular degeneration. Methotrexate, antibodies, dexamethasone, triamcinolone, and other steroid agents may be incorporated into the drug delivery devices to treat uveitis. Anti-proliferative agents, anti-mitotic agents, anti-inflammatory agents, and other medications that would inhibit the spread of lens epithelial cells may be incorporated into the drug delivery devices to treat posterior capsular opacification. Antibiotics such as fluoroquinolones, non-steroidal agents such as ketorolacs, and steroids such as prednisolones may be incorporated into the drug delivery devices for post-op management after cataract surgery to treat infection and inflammation.

While the configurations described above are described in the context of intraocular lens, each configuration may be employed with various other forms of ocular implants intended for implantation in the capsular bag, including capsular tension rings and capsular scaffolds.

While the preferred embodiments of the devices and methods have been described in reference to the environment in which they were developed, they are merely illustrative of the principles of the inventions. The elements of the various embodiments may be incorporated into each of the other species to obtain the benefits of those elements in combination with such other species, and the various beneficial features may be employed in embodiments alone or in combination with each other. Other embodiments and configurations may be devised without departing from the spirit of the inventions and the scope of the appended claims.

All publications, patents, and patent applications mentioned in this specification are herein incorporated by reference to the same extent as if each individual publication, patent, or patent application was specifically and individually indicated to be incorporated by reference.
The following paragraphs describe embodiments of the invention.
1. An ophthalmic implant (11) comprising: an IOL assembly (12) comprising a lens (24), and a haptic (28), the IOL assembly characterized by an anterior surface and a posterior surface, and a circumferential side wall (24C), wherein the posterior surface joins the circumferential side wall in a sharply angular edge (24E), the sharply angular edge defining a plane (P) of the sharply angular edge; and a drug delivery device disposed on the haptic, the drug delivery device having a posterior surface (33P), and a circumferential side wall (33C), wherein the drug delivery device on the haptic does not disrupt formation of a PCO barrier.
2. The ophthalmic implant of paragraph 1, wherein the drug delivery device (30) is configured and disposed on the haptic (28) such that the sharply angular edge (33E) of the drug delivery device (30) is anteriorly displaced from the plane (P).
3. The ophthalmic implant of paragraph 1, wherein the drug delivery device (30) is configured and disposed on the haptic (28) such that the sharply angular edge (33E) of the drug delivery device (30) is posteriorly displaced from the plane (P).
4. The ophthalmic implant of paragraph 1, wherein the drug delivery device (30) is configured and disposed on the haptic (28) such that the sharply angular edge (33E) of the drug delivery device (30) is co-planar with the plane (P).
5. The ophthalmic implant of any one of paragraphs 1-4, wherein the drug delivery device comprises a posterior panel (33) and an anterior panel (36) joined by a pair of posts (35), with an aperture (34) bounded by the posterior panel (33), anterior panel (36) and posts (35), and the haptic is disposed within the aperture to secure the drug delivery device to the haptic.
6. The ophthalmic implant of any one of paragraphs 1-5, wherein a portion of the circumferential side wall (33C) of the posterior panel (33) immediately abuts a portion of the side wall (24C) of the IOL (24).
7. The ophthalmic implant of any one of paragraphs 1-6, wherein the circumferential side wall (33C) of the posterior panel (33) is radially, outwardly displaced from the side wall (24C) of the IOL (24).
8. An ophthalmic implant (11) comprising: an IOL assembly (12) comprising a lens (24), and a haptic (28), the IOL assembly characterized by an anterior surface and a posterior surface, and a circumferential side wall (24C); the haptic (28) is joined to the lens via a haptic base (28B) having a shape and size configured for acceptance of a drug delivery device (30); and a drug delivery device (30) configured for disposition on the haptic (28), the drug delivery device (30) having an aperture (34) configured to accept the haptic base (28B), the aperture (34) being keyed to the shape and size of the haptic base (28B) in a configuration which inhibits rotation of the drug delivery device (30) about the haptic base (28B);
   such that, in an assembled configuration, the drug delivery device (30) is disposed on the haptic base (28B), with the haptic base (28B) occupying the aperture (34).
9. The ophthalmic implant of paragraph 8, wherein the drug delivery device (30) comprises a posterior panel (33) and an anterior panel (36) joined by a pair of posts (35), with an aperture (34) bounded by the posterior panel (33), anterior panel (36) and posts (35).
10. An ophthalmic implant (11) comprising: an IOL assembly (12) comprising a lens (24), and a haptic (28), the IOL assembly characterized by an anterior surface and a posterior surface, and a circumferential side wall (24C); the haptic (28) is joined to the lens (12) via a haptic base (28B) having a shape and size configured for acceptance of a drug delivery device (30); and a drug delivery device (30) configured for disposition on the haptic, the drug delivery device (30) having an aperture (34) configured to accept the haptic base (28B), the aperture (34) being keyed to the shape and size of the haptic base (28B) in a configuration which inhibits rotation of the drug delivery device (30) about the haptic base (28B);
   such that, in an assembled configuration, the drug delivery device (30) is disposed on the haptic base (28B), with the haptic base (28B) occupying the aperture (34).
11. The ophthalmic implant of any one of paragraphs 8-10, wherein the drug delivery device (30) comprises a posterior panel (33) and an anterior panel (36) joined by a pair of posts (35), with an aperture (34) bounded by the posterior panel (33), anterior panel (36) and posts (35).
12. The ophthalmic implant of any one of paragraphs 8-11, wherein the drug delivery device (30) comprises an anterior panel (36) with an anterior surface (36A) and an anterior surface edge (36E), and the anterior surface edge (36E) lies entirely in a plane (A) which is parallel to a major plane of the IOL lens (B).
13. The ophthalmic implant of any one of paragraphs 8-12, wherein the anterior surface (36A) is planar.
14. The ophthalmic implant of any one of paragraphs 8-13, wherein the anterior panel (36) is characterized by a circumferential dimension (40) and a radial dimension (41) and the circumferential dimension (40) is larger than the radial dimension (41).
15. An ophthalmic implant (11) comprising: an IOL assembly (12) comprising a lens (24), and a haptic (28), the IOL assembly characterized by an anterior surface and a posterior surface, and a circumferential side wall (24C); the haptic (28) is joined to the lens via a haptic base (28B) having a shape and size configured for acceptance of a drug delivery device (30);
   a drug delivery device (30) configured for disposition on the haptic (28), the drug delivery device (30) having an aperture (34) configured to accept the haptic base (28B); and the haptic further comprising a first retaining structure, configured to inhibit outward migration of the drug delivery device (30) from the haptic base; wherein in an assembled configuration, first retaining structure (37, 36, 39) obstructs the drug delivery device (30) and inhibits outward migration of the drug delivery device (30) from the haptic base (28B).
16. The ophthalmic implant of paragraph 15, wherein the first retaining structure comprises a detent or shoulder on the haptic, configured to inhibit with outward movement of the drug delivery device (30) along the haptic (28).
17. The ophthalmic implant of any one of paragraphs 15-16, wherein the first retaining structure comprises a notch in the haptic base (28B); and the drug delivery device (30) comprises a second retaining feature comprising a post (35) configured to occupy the notch.
18. The ophthalmic implant of any one of paragraphs 15-17, wherein the drug delivery device (30) comprises a posterior panel (33) and an anterior panel (36) joined by a pair of posts (35), with an aperture (34) bounded by the posterior panel (33), anterior panel (36) and the posts (35); and the drug delivery device (30) is disposed over the haptic with one of the posts (35) disposed within the notch.
19. The ophthalmic implant of any one of paragraphs 8-18, wherein the drug delivery device (30) is configured and disposed on the haptic (28) such that the sharply angular edge (33E) of the drug delivery device (30) is anteriorly displaced from the plane (P).
20. The ophthalmic implant of any one of paragraphs 8-18, wherein the drug delivery device (30) is configured and disposed on the haptic (28) such that the sharply angular edge (33E) of the drug delivery device (30) is posteriorly displaced from the plane (P).
21. The ophthalmic implant of any one of paragraphs 8-18, wherein the drug delivery device (30) is configured and disposed on the haptic (28) such that the sharply angular edge (33E) of the drug delivery device (30) is co-planar with the plane (P).
22. The ophthalmic implant of paragraphs 2 or 19, wherein the posterior-most surface (30P) of the drug delivery device (30) is anteriorly displaced from the plane (P) by 0.1 to 1 millimeters.
23. An ophthalmic implant (11) comprising: an IOL assembly (12) comprising a lens (24), and a haptic (28), the IOL assembly characterized by an anterior surface (24A) and a posterior surface (24P), and a circumferential side wall (24C), wherein the posterior surface joins the circumferential side wall in a sharply angular edge (24E), the sharply angular edge (24E) defining a plane (P) of the sharply angular edge (24E); and a drug delivery device (30) disposed on the haptic (28), the drug delivery device (30) configured and disposed on the haptic (28) such that a posterior-most surface (30P) of the drug delivery device (30) is anteriorly displaced from the plane (P), whereby the presence of the drug delivery device (30) on the haptic does (28) not disrupt formation of a PCO barrier.
24. The ophthalmic implant of paragraph 23, wherein the posterior-most surface (30P) of the drug delivery device (30) is anteriorly displaced from the plane (P) by 0.1 to 1 millimeters.
25. The ophthalmic implant of any one of paragraphs 23-24, wherein the haptic (28) is joined to the lens (24) at a joint, and the posterior-most extent of the joint between the haptic and the lens is displaced/offset anteriorly from the sharply angular edge (24E).
26. The ophthalmic implant of any one of paragraphs 23-25, wherein the haptic is joined to the lens (24) at a joint, and the posterior-most extent of the joint is displaced/offset anteriorly from the sharply angular edge, whereby the presence of the haptic does not disrupt formation of a PCO barrier between the IOL sharp edge and the posterior portion of the capsule.
27. The ophthalmic implant of any one of paragraphs 23-26, wherein the sharply angular edge is characterized by an inside angle and a corner radius, between the posterior panel surface and the circumferential side wall of the posterior panel is in the range of 80° to 100°, and the comer radius of the sharply angular edge is less than 500 pm, and preferably less than 60 pm.
28. The ophthalmic implant of any one of paragraphs 1-27, wherein the drug delivery device comprises one or more therapeutic agents.
29. The ophthalmic implant of paragraph 28, wherein the one or more therapeutic agents comprises a beta blocker, an alpha agonist, a ROCK Inhibitor, an adenosine receptor agonist, a carbonic anhydrase inhibitor, an adrenergic or cholinergic receptor activating agent, a prostaglandin analogue, a steroid, an aptamer, an antibody, a complement factor, an anti-oxidant, an anti-inflammatory agent, an anti-proliferative agents, an anti-mitotic agents, an antibiotic, or a combination thereof.
30. The ophthalmic implant of any one of paragraphs 28-29, wherein the one or more therapeutic agents comprises or consists of a prostaglandin analogue.
31. A method of treating an eye disease in a patient in need thereof, the method comprising: implanting an ophthalmic implant according to any one of paragraphs 1-30 within a capsular bag of the eye of the patient, thereby treating the eye disease in the patient.
32. The method of paragraph 31, further comprising allowing the capsular bag to approximate the IOL.
33. The method of any one of paragraphs 31-32, further comprising forming a PCO barrier between the posterior portion of the capsular bag and the sharply angular edge.
34. The method of any one of paragraphs 31-33, wherein the eye disease comprises glaucoma, wet macular degeneration, dry macular degeneration, uveitis, an infection or inflammation of the eye, or a combination thereof.
35. The method of any one of paragraphs 31-34, wherein the implanting comprises:
   folding or rolling the ophthalmic implant into a small diameter configuration into an injector;
   inserting the injector through a small slit between the cornea and sclera of the eye; and
   pushing the ophthalmic implant from the injector into a tissue of the eye.

## Claims

1. An ophthalmic implant (11) comprising: an IOL assembly (12) comprising:
a lens (24), and a haptic (28), the IOL assembly **characterized by** an anterior surface and a posterior surface, and a circumferential side wall (24C); the haptic (28) is joined to the lens via a haptic base (28B) having a shape and size configured for acceptance of a drug delivery device (30); and
a drug delivery device (30) configured for disposition on the haptic (28), the drug delivery device (30) having an aperture (34) configured to accept the haptic base (28B), the aperture (34) being keyed to the shape and size of the haptic base (28B) in a configuration which inhibits rotation of the drug delivery device (30) about the haptic base (28B);
such that, in an assembled configuration, the drug delivery device (30) is disposed on the haptic base (28B), with the haptic base (28B) occupying the aperture (34).

2. The ophthalmic implant of claim 1, wherein the drug delivery device (30) comprises a posterior panel (33) and an anterior panel (36) joined by a pair of posts (35), with an aperture (34) bounded by the posterior panel (33), anterior panel (36) and posts (35).

3. An ophthalmic implant (11) comprising:
an IOL assembly (12) comprising a lens (24), and a haptic (28), the IOL assembly **characterized by** an anterior surface and a posterior surface, and a circumferential side wall (24C); the haptic (28) is joined to the lens (12) via a haptic base (28B) having a shape and size configured for acceptance of a drug delivery device (30); and
a drug delivery device (30) configured for disposition on the haptic, the drug delivery device (30) having an aperture (34) configured to accept the haptic base (28B), the aperture (34) being keyed to the shape and size of the haptic base (28B) in a configuration which inhibits rotation of the drug delivery device (30) about the haptic base (28B);
such that, in an assembled configuration, the drug delivery device (30) is disposed on the haptic base (28B), with the haptic base (28B) occupying the aperture (34).

4. The ophthalmic implant of any one of claims 1-3, wherein the drug delivery device (30) comprises a posterior panel (33) and an anterior panel (36) joined by a pair of posts (35), with an aperture (34) bounded by the posterior panel (33), anterior panel (36) and posts (35).

5. The ophthalmic implant of any one of claims 1-4, wherein the drug delivery device (30) comprises an anterior panel (36) with an anterior surface (36A) and an anterior surface edge (36E), and the anterior surface edge (36E) lies entirely in a plane (A) which is parallel to a major plane of the **IOL** lens (B).

6. The ophthalmic implant of any one of claims 1-5, wherein the anterior surface (36A) is planar.

7. The ophthalmic implant of any one of claims 1-6, wherein the anterior panel (36) is **characterized by** a circumferential dimension (40) and a radial dimension (41) and the circumferential dimension (40) is larger than the radial dimension (41).

8. An ophthalmic implant (11) comprising:
**an IOL** assembly (12) comprising a lens (24), and a haptic (28), the **IOL** assembly **characterized by** an anterior surface and a posterior surface, and a circumferential side wall (24C); the haptic (28) is joined to the lens via a haptic base (28B) having a shape and size configured for acceptance of a drug delivery device (30);
a drug delivery device (30) configured for disposition on the haptic (28), the drug delivery device (30) having an aperture (34) configured to accept the haptic base (28B); and the haptic further comprising a first retaining structure, configured to inhibit outward migration of the drug delivery device (30) from the haptic base;
wherein in an assembled configuration, first retaining structure (37, 36, 39) obstructs the drug delivery device (30) and inhibits outward migration of the drug delivery device (30) from the haptic base (28B).

9. The ophthalmic implant of claim 8, wherein the first retaining structure comprises a detent or shoulder on the haptic, configured to inhibit with outward movement of the drug delivery device (30) along the haptic (28).

10. The ophthalmic implant of claim 8 or claim 9, wherein the first retaining structure comprises a notch in the haptic base (28B); and the drug delivery device (30) comprises a second retaining feature comprising a post (35) configured to occupy the notch.

11. The ophthalmic implant of any one of claims 8-10, wherein the drug delivery device (30) comprises a posterior panel (33) and an anterior panel (36) joined by a pair of posts (35), with an aperture (34) bounded by the posterior panel (33), anterior panel (36) and the posts (35); and the drug delivery device (30) is disposed over the haptic with one of the posts (35) disposed within the notch.
